# EUROPÄISCHE PATENTSCHRIFT

(11) **EP 1 402 267 B1**
(45) Veröffentlichungstag und Bekanntmachung des Hinweises auf die Patenterteilung: **21.12.2005**
(21) Anmeldenummer: 02735421.6
(22) Anmeldetag: 17.06.2002
(51) Int. Cl.: G01N 33/68, G01N 33/569, A61P 29/00

(54) **CHP ALS MARKER FÜR SEPSIS**
CHP FOR USE AS MARKER FOR SEPSIS
UTILISATION DE LA PROTEINE CHP COMME MARQUEUR POUR LA SEPTICEMIE

(30) Priorität: 02.07.2001 DE 10131922
(43) Veröffentlichungstag der Anmeldung: 31.03.2004
(73) Patentinhaber: B.R.A.H.M.S Aktiengesellschaft, 16761 Hennigsdorf (DE)
(72) Erfinder: BERGMANN, Andreas, 12351 Berlin (DE); STRUCK, Joachim, 12161 Berlin (DE); ÜHLEIN, Monika, 10407 Berlin (DE)
(74) Vertreter: Andrae, Steffen
(86) Internationale Anmeldenummer: PCT/EP2002/006660
(87) Internationale Veröffentlichungsnummer: WO 2003/005035

(56) Entgegenhaltungen:
- WO-A-00/22439
- WO-A-99/37790
- LIN X ET AL: "Inhibition of calcineurin phosphatase activity by a calcineurin B homologous protein." THE JOURNAL OF BIOLOGICAL CHEMISTRY., Bd. 274, Nr. 51, 17. Dezember 1999 (1999-12-17), Seiten 36125-36131, XP002213293 UNITED STATES ISSN: 0021-9258 in der Anmeldung erwähnt -& DATABASE SWISS-PROT [Online] ID: CA22_HUMAN , 15. Juli 1998 (1998-07-15) "Calcium-binding protein p22 (CHP)" Database accession no. Q99653 XP002213294

### SEQUENCE LISTING

<110> B.R.A.H.M.S Aktiengesellschaft
<120> Verwendungen des Calcineurin B Homologen Proteins (CHB) für die Diagnose und Therapie von Entzündungserkrankungen und Sepsis
<130> 3536 PCT AS
<140>
   <141>
<150> DE 101 31 922.3
   <151> 2001-07-02
<160> 6
<170> PatentIn Ver. 2.1
<210> 1
   <211> 10
   <212> PRT
   <213> Primat (Pavian)
<400> 1
   Leu Tyr Ser Arg Phe Thr Ser Leu Asp Lys
   1               5                   10
<210> 2
   <211> 12
   <212> PRT
   <213> Primat (Pavian)
<400> 2
   Lys Glu Thr Gly Phe Ser His Ser Gln Ile Thr Arg
   1               5                   10
<210> 3
   <211> 13
   <212> PRT
   <213> Primat (Pavian)
<400> 3
   Thr Leu Ala His Phe Arg Pro Ile Glu Asp Asn Glu Lys
   1               5                   10
<210> 4
   <211> 14
   <212> PRT
   <213> Primat (Pavian)
<400> 4
   Ala Ser Thr Leu Leu Arg Asp Glu Glu Leu Glu Glu Ile Lys
   1               5                   10
<210> 5
   <211> 20
   <212> PRT
   <213> Primat (Pavian)
<400> 5
   Ile Ile Asn Ala Phe Phe Pro Glu Gly Glu Asp Gln Val Asn Phe Arg
   1               5                   10                  15
   Gly Phe Met Arg
               20
<210> 6
   <211> 194
   <212> PRT
   <213> Homo sapiens
<300>
   <301> Lin, Xia
   <302> A calcineurin homologous protein inhibits GTPase-stimulated Na-H exchange
   <303> Proc. Natl. Acad. Sci. U.S.A.
   <304> 1996
   <305> vol.93
   <306> 12631-12636
<400> 6
   Gly Ser Arg Ala Ser Thr Leu Leu Arg Asp Glu Glu Leu Glu Glu Ile
   1               5                   10                  15
   Lys Lys Glu Thr Gly Phe Ser His Ser Gln Ile Thr Arg Leu Tyr Ser
               20                  25                  30
   Arg Phe Thr Ser Leu Asp Lys Gly Glu Asn Gly Thr Leu Ser Arg Glu
           35                  40                  45
   Asp Phe Gln Arg Ile Pro Glu Leu Ala Ile Asn Pro Leu Gly Asp Arg
       50                  55                  60
   Ile Ile Asn Ala Phe Phe Pro Glu Gly Glu Asp Gln Val Asn Phe Arg
   65                  70                  75                  80
   Gly Phe Met Arg Thr Leu Ala His Phe Arg Pro Ile Glu Asp Asn Glu
                   85                  90                  95
   Lys Ser Lys Asp Val Asn Gly Pro Glu Pro Leu Asn Ser Arg Ser Asn
               100                 105                 110
   Lys Leu His Phe Ala Phe Arg Leu Tyr Asp Leu Asp Lys Asp Glu Lys
           115                 120                 125
   Ile Ser Arg Asp Glu Leu Leu Gln Val Leu Arg Met Met Val Gly Val
       130                 135                 140
   Asn Ile Ser Asp Glu Gln Leu Gly Ser Ile Ala Asp Arg Thr Ile Gln
   145                 150                 155                 160
   Glu Ala Asp Gln Asp Gly Asp Ser Ala Ile Ser Phe Thr Glu Phe Val
                   165                 170                 175
   Lys Val Leu Glu Lys Val Asp Val Glu Gln Lys Met Ser Ile Arg Phe
               180                 185                 190
   Leu His

## Beschreibung

Die vorliegende Erfindung betrifft neue Verwendungen des sogenannten Calcineurin B Homologen Proteins (CHP) für die medizinische Diagnostik von Sepsis. Sie beruht auf dem erstmaligen Nachweis stark erhöhter Konzentrationen von CHP in Lebergewebe von Primaten, bei denen experimentell durch Toxinverabreichunge eine Sepsis bzw. systemische Entzündung hervorgerufen wurde.

Die vorliegende Erfindung hat ihren Ursprung in intensiven Forschungsarbeiten der Anmelderin im Zusammenhang mit weiteren Verbesserungen der Diagnose und Therapie von Entzündungen und Infektionen, insbesondere von Entzündungen infektiöser Ätiologie und Sepsis.

Als Entzündungen (Inflammationen) werden ganz allgemein bestimmte physiologische Reaktionen eines Organismus auf verschiedenartige äußere Einwirkungen wie z.B. Verletzungen, Verbrennungen, Allergene, Infektionen durch Mikroorganismen wie Bakterien und Pilze und Viren, auf Fremdgewebe, die Abstoßungsreaktionen auslösen, oder auf bestimmte entzündungsauslösende endogene Zustände des Körpers, z.B. bei Autoimmunerkrankungen und Krebs, bezeichnet. Entzündungen können als harmlose, lokal begrenzte Reaktionen des Körpers auftreten, sind jedoch auch typische Merkmale zahlreicher ernster chronischer und akuter Erkrankungen von einzelnen Geweben, Organen, Organ- und Gewebsteilen.

Lokale Entzündungen sind dabei meist Teil der gesunden Immunreaktion des Körpers auf schädliche Einwirkungen, und damit Teil des lebenserhaltenden Abwehrmechanismus des Organismus. Wenn Entzündungen jedoch Teil einer fehlgeleiteten Reaktion des Körpers auf bestimmte endogene Vorgänge wie z.B. bei Autoimmunerkrankungen sind und/oder chronischer Natur sind, oder wenn sie systemische Ausmaße erreichen, wie beim systemischen Inflammationssyndrom (Systemic Inflammatory Response Syndrome, SIRS) oder bei einer auf infektiöse Ursachen zurückzuführenden schweren Sepsis, geraten die für Entzündungsreaktioner typischen physiologischen Vorgänge außer Kontrolle und werden zum eigentlichen, häufig lebensbedrohlichen Krankheitsgeschehen.

Es ist heute bekannt, dass die Entstehung und der Verlauf von entzündlichen Prozessen von einer beträchtlichen Anzahl von Substanzen, die überwiegend proteinischer bzw. peptidischer Natur sind, gesteuert werden bzw. von einem mehr oder weniger zeitlich begrenzten Auftreten bestimmter Biomoleküle begleitet sind. Zu den an Entzündungsreaktionen beteiligten endogenen Substanzen gehören insbesondere solche, die zu den Cytokinen, Mediatoren, vasoaktiven Substanzen, Akutphasenproteinen und/oder hormonellen Regulatoren gezählt werden können. Die Entzündungsreaktion stellt eine komplexe physiologische Reaktion dar, an der sowohl das Entzündungsgeschehen aktivierende endogene Substanzen (z.B. TNF-α) als auch desaktivierende Substanzen (z.B. Interleukin-10) beteiligt sind.

Bei systemischen Entzündungen wie im Falle einer Sepsis bzw. des septischen Schocks weiten sich die entzündungsspezifischen Reaktionskaskaden unkontrolliert auf den gesamten Körper aus und werden dabei, im Sinne einer überschießenden Immunantwort, lebensbedrohlich. Zu den gegenwärtigen Kenntnissen über das Auftreten und die möglichen Rolle einzelner Gruppen endogener entzündungsspezifischer Substanzen wird beispielsweise verwiesen auf A. Beishuizen, et al., "Endogenous Mediators in Sepsis and Septic Shock", Advances in Clinical Chemistry, Vol. 33, 1999, 55-131; und C.Gabay, et al., "Acute Phase Proteins and Other Systemic Responses to Inflammation", The New England Journal of Medicine, Vol.340, No.6, 1999, 448-454. Da sich das Verständnis von Sepsis und verwandten systemischen entzündlichen Erkrankungen, und damit auch die anerkannten Definitionen, in den letzten Jahren gewandelt haben, wird außerdem verwiesen auf K.Reinhart, et al., "Sepsis und septischer Schock", in: Intensivmedizin, Georg Thieme Verlag, Stuttgart.New York, 2001, 756-760; wo eine moderne Definition des Sepsis-Begriffes gegeben wird. Im Rahmen der vorliegenden Anmeldung werden die Begriffe Sepsis bzw. entzündliche Erkrankungen in Anlehnung an die Definitionen verwendet, wie sie den genannten drei Literaturstellen entnommen werden können.

Während wenigstens im europäischen Raum die durch eine positive Blutkultur nachweisbare systemische bakterielle Infektion lange den Sepsisbegriff prägte, wird die Sepsis heute in erster Linie als systemische Entzündung verstanden, die infektiöse Ursachen hat, als Krankheitsgeschehen jedoch große Ähnlichkeiten mit systemischen Entzündungen aufweist, die durch andere Ursachen ausgelöst werden. Dem genannten Wandel des Sepsis-Verständnisses entsprechen Veränderungen der diagnostischen Ansätze. So wurde der direkte Nachweis bakterieller Erreger durch komplexe Überwachungen physiologischer Parameter und in jüngerer Zeit insbesondere auch durch den Nachweis bestimmter am Sepsisgeschehen bzw. am Entzündungsgeschehen beteiligter endogener Substanzen, d.h. spezifischer "Biomarker", ersetzt bzw. ergänzt.

Von der großen Zahl von Mediatoren und Akutphasenproteinen, von denen man weiß, dass sie an einem Entzündungsgeschehen beteiligt sind, eignen sich dabei für diagnostische Zwecke insbesondere solche, deren Auftreten sehr spezifisch für entzündliche Erkrankungen bzw. bestimmte Phasen entzündlicher Erkrankungen ist, deren Konzentrationen sich drastisch und diagnostisch signifikant verändern und die außerdem die für Routinebestimmungen erforderlichen Stabilitäten aufweisen und hohe Konzentrationswerte erreichen. Für diagnostische Zwecke steht dabei die zuverlässige Korrelation von Krankheitsgeschehen (Entzündung, Sepsis) mit dem jeweiligen Biomarker im Vordergrund, ohne dass dessen Rolle in der komplexen Kaskade der am Entzündungsgeschehen beteiligten endogenen Substanzen bekannt sein muss.

Eine bekannte, als Sepsis-Biomarker besonders geeignete endogene Substanz ist Procalcitonin. Procalcitonin ist ein Prohormon, dessen Serum-Konzentrationen unter den Bedingungen einer systemischen Entzündung infektiöser Ätiologie (Sepsis) sehr hohe Werte erreichen, während es bei Gesunden so gut wie nicht nachweisbar ist. Hohe Werte an Procalcitonin werden außerdem in einem relativ frühen Stadium einer Sepsis erreicht, so dass sich die Bestimmung von Procalcitonin auch zur Früherkennung einer Sepsis und zur frühen Unterscheidung einer infektiös bedingten Sepsis von schweren Entzündungen, die auf anderen Ursachen beruhen, eignet. Die Bestimmung von Procalcitonin als Sepsismarker ist Gegenstand der Veröffentlichung M.Assicot, et al., "High serum procalcitonin concentrations in patients with sepsis and infection", The Lancet, vol.341, No.8844, 1993, 515-518; und der Patente DE 42 27 454 C2 bzw. EP 0 656 121 B1 bzw. US 5,639,617. In den letzten Jahren ist die Zahl der Veröffentlichungen zum Thema Procalcitonin stark angestiegen. Stellvertretend für zusammenfassende Veröffentlichungen aus jüngerer Zeit wird daher noch verwiesen auf W.Karzai et al., "Procalcitonin - A New Indicator of the Systemic Response to Severe infection", Infection, Vol. 25, 1997, 329-334; und M.Oczenski et al., "Procalcitonin: a new parameter for the diagnosis of bacterial infection in the peri-operative period", European Journal of Anaesthesiology 1998, 15, 202-209; sowie ferner H.Redl, et al., "Procalcitonin release patterns in a baboon model of trauma and sepsis: Relationship to cytokines and neopterin", Crit Care Med 2000, Vol.28. No.11, 3659-3663; und H.Redl, et al., "Non-Human Primate Models of Sepsis", in: Sepsis 1998; 2:243-253; und die darin zitierten weiteren Literaturstellen.

Die Verfügbarkeit des Sepsismarkers Procalcitonin hat der Sepsisforschung starke Impulse gegeben, und es werden gegenwärtig intensive Anstrengungen unternommen, weitere Biomarker zu finden, die die Procalciconin-Bestimmung ergänzen können und/oder zusätzliche Informationen für Zwecke der Feindiagnostik bzw. Differentialdiagnostik zu liefern vermögen. Erschwert wird die Suche nach potentiellen neuen Sepsis-Biomarkern allerdings dadurch, dass häufig noch sehr wenig oder nichts über die genaue Funktion bzw. über die genauen Gründe für das Auftreten bestimmter endogener Substanzen, die am Entzündungs- oder Sepsisgeschehen beteiligt sind, bekannt ist.

Die Ergebnisse der experimentellen Überprüfung eines fruchtbaren rein hypothetischen Ansatzes zur Ermittlung weiterer potentieller Sepsismarker finden sich in DE 198 47 690 A1 bzw. WO 00/22439. Dort wird gezeigt, dass bei Sepsis nicht nur die Konzentration des Prohormons Procalcitonin erhöht ist, sondern auch für andere Substanzen, die zu den Peptid-Prohormonen gerechnet werden können, signifikant erhöhte Konzentrationen beobachtet werden können. Während das beschriebene Phänomen gut dokumentiert ist, sind die Ursachen für den Anstieg der Konzentrationen von Prohormonen bei Sepsis weiterhin weitgehend ungeklärt.

In der vorliegenden Anmeldung wird nunmehr Ergebnisse eines anderen, rein experimentellen Ansatzes für die Suche nach weiteren entzündungs- bzw. sepsisspezifischen Biomolekülen berichtet. Auch diese experimentellen Untersuchungen nehmen ihren Ausgang bei der Bestimmung von Procalcitin im Zusammenhang mit systemischen entzündlichen Reaktionen infektiöser Ätiologie. So war sehr früh beobachtet worden, dass bei Sepsis das Procalcitonin offensichtlich nicht auf die gleiche Weise gebildet wird, wie dann, wenn es Vorläufer für das Hormon Calcitonin ist. So wurden hohe Procalcitoninspiegel auch bei Patienten beobachtet, denen die Schilddrüse entfernt worden war. Deshalb kann die Schilddrüse nicht dasjenige Organ sein, in dem Procalcitonin bei Sepsis gebildet bzw. ausgeschüttet wird. In den Veröffentlichungen H.Redl, et al., "Procalcitonin release patterns in a baboon model of trauma and sepsis: Relationship to cytokines and neopterin", Crit Care Med 2000, Vol. 28. No. 11, 3659-3663; und H.Redl, et al., "Non-Human Primate Models of Sepsis", Sepsis 1998; 2:243-253; werden die Ergebnissen von experimentellen Untersuchungen berichtet, die der Klärung der Bildung von Procalcitonin bei Sepsis dienen sollten. In den genannten Arbeiten wird durch Endotoxinverabreichung an Primaten (Paviane) eine künstliche Sepsis erzeugt, und es wird bestimmt, bei welchen experimentell erzeugten Zuständen die höchsten Procalcitoninkonzentrationen im Blut erreicht werden. Eine Weiterentwicklung des in den genannten Arbeiten beschriebene Versuchstiermodells dient im Rahmen der vorliegenden Anmeldung dazu, neue endogene sepsisspezifische Biomarker von peptischer bzw. proteinischer Natur zu ermitteln, deren Auftreten für Sepsis oder bestimmte Formen von Sepsis charakteristisch ist und die daher eine spezifische Sepsisdiagnose ermöglichen. Das Primatenmodell wurde dabei aufgrund der sehr großen Ähnlichkeit der Physiologie von Primaten und Menschen und der hohen Kreuzreaktivität mit vielen therapeutischen und diagnostischen humanen Reagenzien gewählt.

Da die bei Entzündungen gebildeten endogenen Substanzen Teil der komplexen Reaktionskaskade des Körpers sind, sind derartige Substanzen außerdem nicht nur von diagnostischem Interesse, sondern es wird gegenwärtig auch mit erheblichem Aufwand versucht, durch Beeinflussung der Entstehung und/oder der Konzentration einzelner derartiger Substanzen therapeutisch in das Entzündungsgeschehen einzugreifen, um die zum Beispiel bei Sepsis beobachtete systemische Ausbreitung der Entzündung möglichst frühzeitig zu stoppen. In diesem Sinne sind nachweislich am Entzündungsgeschehen beteiligte endogene Substanzen auch als potentielle therapeutische Targets anzusehen. Versuche, ansetzend an bestimmten Mediatoren des Entzündungsgeschehens dieses positiv therapeutisch zu beeinflussen, sind beschrieben beispielsweise in E.A.Panacek, "Anti-TNF strategies", Journal für Anästhesie und Intensivbehandlung; Nr.2, 2001, 4-5; T.Calandra, et al., "Protection from septic shock by neutralization of macrophage migration inhibitory factor", Nature Medicine, Vol.6, No.2, 2000, 164-170; oder K.Garber, "Protein C may be sepsis solution", Nature Biotechnology, Vol.18, 2000, 917-918. Diese therapeutischen Ansätze laufen darauf hinaus, die Konzentrationen entzündungsfördernder Substanzen zu senken bzw. die Entstehung derartiger Substanzen zu hemmen, und zwar insbesondere unter Verwendung von spezifischen Antikörpern (gegen TNF-α bzw. MIF; vgl. E.A.Panacek, "Anti-TNF strategies", Journal für Anästhesie und Intensivbehandlung; Nr.2, 2001, 4-5; T.Calandra, et al., "Protection from septic shock by neutralization of macrophage migration inhibitory factor", Nature Medicine, Vol.6, No.2, 2000, 164-170) bzw. die Konzentration von hemmend in die Entzündungskaskade eingreifenden endogenen Substanzen (Protein C; K.Garber, "Protein C may be sepsis solution", Nature Biotechnology, Vol.18, 2000, 917-918) zu erhöhen. In der letztgenannten Veröffentlichung findet sich ein Überblick über derartige, leider bisher meist wenig erfolgreiche Versuche, das Entzündungsgeschehen unter Beeinflussung ausgewählter endogener Target-Moleküle therapeutisch zu beeinflussen. Angesichts der bisherigen eher enttäuschenden therapeutischen Ansätze besteht ein hohes Interesse daran, weitere möglichst entzündungs- bzw. sepsisspezifische endogene Biomoleküle zu identifizieren, die auch als therapeutische Targets neue Erfolgsaussichten für die Entzündungsbekämpfung eröffnen.

Die vorliegende Erfindung beruht darauf, dass sich in Primaten und Menschen bei infektiös bedingten Entzündungen das sogenannte Calcineurin B Homologe Protein (CHP) in beträchtlichen Konzentrationen nachweisen läßt, und zwar im Unterschied zu Gesunden, bei denen es nicht oder nur in Konzentrationen an der analytischen Nachweisgrenze gefunden wird, was dieses Protein CHP für die Entzündungsdiagnostik/Sepsisdiagnostik geeignet macht.

Die Verwendungen, die sich aufgrund des erstmals nachgewiesenen Auftretens von CHP bei der experimentellen Simulation einer Sepsis für diagnostische Zwecke ergeben, werden in allgemeiner Form in Anspruch 1 beansprucht.

Die Ansprüche 2 bis 4 betreffen ebenfalls diagnostische Verwendungen bzw. Verfahren.

Wie nachfolgend im experimentellen Teil noch näher ausgeführt wird, beruht die Erfindung darauf, dass nach experimenteller Auslösung einer künstlichen Sepsis in Pavianen durch Endotoxinverabreichung (LPS aus Salmonella Typhimurium) und 2D-gelektrophoretischer Aufarbeitung von Lebergewebe der behandelten Tiere als eines der nur bei den behandelten Tieren identifizierbares Peptid- bzw. Proteinprodukt CHP gefunden werden konnte. Zu seiner Identifizierung wurde ein neuer Proteinspot, der nur bei den behandelten Tieren gefunden wurde und gemäß Gelektrophorese ein Molekulargewicht von etwa 22900 ± 700 Dalton und einen isoelektrischen Punkt von ca. 6,2 aufwies, aus dem Elektrophoresegel isoliert und durch Trypsinverdauung in Bruchstücke zerlegt, die auf an sich bekannte Weise massenspektrometrisch analysiert und durch Vergleich mit bekannten trypsinbehandelten Proteine identifiziert wurden.

Bei der Massenanalyse des tryptischen Verdaus des neuen Proteinspots wurden Bruchstücke mit folgenden Massen (M/Z) gefunden: 1169,52; 1469,50; 1550,56; 1569,7; 1645,6; 1920,81; 2386,78. Zwei der 7 Massen (1169,52 und 1920,81) konnten keinen Massen bekannter trypsinbehandelter Proteine zugeordnet werden. Für die restlichen 5 Massen ergab sich die folgende Analyse und Zuordnung zu der bekannten Sequenz von CHP (vgl. SEQ ID NO:6; Datenbank SWISS-PROT: Entry CA22_Human; Accession number Q99653; Lin X., Barber D.L. "A calcineurin homologous protein inhibits GTPase-stimulated na-H exchange", Proc.Natl.Acad.Sci.U.S.A. 93:12636(1996)):

Die Masse 1469,50 entspricht der Sequenz L-Y-S-R-F-T-S-L-D-K (SEQ ID NO:1). An den Aminosäure S (Serin, 2faches Auftreten) und T (Threonin) ist die Sequenz durch jeweils eine Phosphatgruppe modifiziert. Das theoretische Molekulargewicht dieses Fragments beträgt 1469,499. In der Aminosäuresequenz von CHP (ohne den bei Lin X., supra, zusätzlich aufgeführten Methioninrest) findet sich diese Sequenz an den Positionen 31-40 wieder.

Die Masse 1550,56 entspricht der Sequenz K-E-T-G-F-S-H-S-Q-I-T (SEQ ID NO:2), ergänzt um 2 Phosphatgruppen. Das theoretische Molekulargewicht dieses Fragments beträgt 1550,564. In der Aminosäuresequenz von CHP (ohne den bei Lin X., supra, zusätzlich aufgeführten Methioninrest) findet sich diese Sequenz an den Positionen 19-30 wieder.

Die Masse 1569,7 entspricht der Sequenz T-L-A-H-F-R-P-I-E-D-N-E-K (SEQ ID NO:3). Das theoretische Molekulargewicht dieses Fragments beträgt 1569,71. In der Aminosäuresequenz von CHP (ohne den bei Lin X., supra, zusätzlich aufgeführten Methioninrest) findet sich diese Sequenz an den Positionen 86-98 wieder.

Die Masse 1645,6 entspricht der Sequenz A-S-T-L-L-R-D-E-E-I-K (SEQ ID NO:4). Das theoretische Molekulargewicht dieses Fragments beträgt 1645,597. In der Aminosäuresequenz von CHP (ohne den bei Lin X., supra, zusätzlich aufgeführten Methioninrest) findet sich diese Sequenz an den Positionen 5-18 wieder.

Die Masse 2386,78 entspricht der Sequenz I-I-N-A-F-F-P-E-G-E-D-Q-V-N-F-R-G-F-M-R (SEQ ID NO:5). Das theoretische Molekulargewicht dieses Fragments beträgt 2386,78. In der Aminosäuresequenz von CHP (ohne den bei Lin X., supra, zusätzlich aufgeführten Methioninrest) findet sich diese Sequenz an den Positionen 66-85 wieder.

Eine solche Übereinstimmung gilt nach anerkannten Interpretationsgrundsätzen als sichere Identifizierung des Produkts aus dem Proteinspot als CHP. Das Molekulargewicht von CHP (194 Aminosäuren) beträgt 22 325 Dalton.

Die Identifizierung des Auftretens von CHP bei Sepsis in der Leber ist von hohem wissenschaftlichen, diagnostischen und therapeutischen Interesse.

Das humane Protein CHP wurde erstmals im Jahr 1996 beschrieben (X.Lin and D.L.Barber, A calcineurin homologous protein inhibits GTPase-stimulated Na-H exchange, Proc.Natl.Acad.Sci.USA, 93:12631-12636, 1996) und erwies sich als im wesentlichen identisch mit dem kurz zuvor im gleichen Jahre als Ca²⁺-bindendes Protein beschriebenen Ratten-Protein p22 (M.R.Barroso et al., A Novel Ca²⁺-bindung Protein, p22, Is Required for Constitutive Membrane Traffic, J.Biol.Chem., 271:10183-10187, 1996). Die Bezeichnung Calcineurin B Homologes Protein (CHP) verweist auf die hohe Ähnlichkeit dieses Proteins mit der regulatorischen B-Untereinheit von Calcineurin (65%). Es besteht außerdem eine hohe Ähnlichkeit von CHP mit einem weiteren Ca²⁺-bindenden Protein, Calmodulin (59%).

CHP wird in vielen humanen Geweben exprimiert und erwies sich als essentieller Cofaktor für die Regulierung von NHE1, einem ubiquitär exprimierten Na⁺/H⁺-Exchanger, der bei seiner Aktivierung den intrazellulären pH-Wert erhöht, was eine Aktivierung der Zellvermehrung, Zelldifferenzierung und neoplastische Transformationen bewirken kann. Für die NHE1-Regulierung während der Zellteilung ist der Phosphorylierungsgrad von CHP wichtig. Es hat sich ferner gezeigt, dass eine transiente Überexpression von CHP die serum- und GTPase-stimulierten NHE1-Aktivitäten inhibiert.

Weitere Untersuchungen zur physiologischen Rolle von CHP bestätigten dessen Bedeutung für die NHE-Regulierung (T.Pang et al., Calcineurin Homologous Protein as an Essential Cofactor for Na⁺/H⁺ Exchangers, J.Biol.Chem., 276:17367-17372, 2001). Sie ergaben ferner, dass CHP die Calcineurin Aktivität inhibiert (X.Lin et al., Inhibition of Calcineurin Phosphatase Activity by a Calcineurin B Homologous Protein, J.Biol.Chem., 274:36125-36131, 1999). Dieser Befund ist von hohem Interesse, weil Calcineurin u.a. eine wichtige Rolle bei der T-Zell-Aktivierung und damit auch bei der Auslösung einer Immunantwort spielt. Calcineurin ist aus diesem Grund auch Target der immunsuppressiven pharmazeutischen Wirkstoffe Cyclosporin A und FK506. Das Protein CHP erweist sich im Lichte dieser Ergebnisse als vorher nicht erkannter endogener Inhibitor der Calcineurin-Wirkung bzw. der Aktivierung des Immunsystems.

Es ist bisher nicht bekannt, das die physiologischen Konzentrationen von CHP bei irgendwelchen Krankheiten signifikant verändert sind und daher eine Bestimmung der CHP-Konzentrationen unter diagnostischen Gesichtspunkten von Interesse sein könnte.

In der WO 99/37790 wird ein mit CHP verwandtes Protein (CAB genannt) mit nur 27%iger Identität mit CHP beschrieben, für das verschiedene diagnostische und therapeutische Verwendungsmöglichkeiten vorgeschlagen werden, ohne dass derartige Vorschläge durch experimentelle Befunde untermauert würden.

Der erfindungsgemäße Nachweis von vergleichsweise hohen CHP-Konzentrationen in der Leber von Primaten, bei denen durch Toxinverabreichung eine künstliche Sepsis ausgelöst wurde, bei gleichzeitiger Unmöglichkeit, CHP in ansonsten völlig gleich behandelten Proben von Kontrolltieren zu erkennen, ist hoch signifikant. Da das Auftreten nur bei den behandelten Tieren zu beobachten war, und zwar bereits relativ kurze Zeit nach der Sepsisauslösung durch Toxinverabreichung, ist es möglich, diese Tatsache zur Schaffung eines vielversprechenden diagnostischen Sepsis-, Infektions- und Entzündungs-Nachweisverfahrens durch Bestimmung von CHP zu nutzen. Von besonderem Interesse ist eine möglich erscheinende Eignung von CHP als Prognosemarker für Sepsis.

Die Bestimmung von CHP kann dabei nach irgendeinem beliebigen geeigneten Nachweisverfahren erfolgen, wobei jedoch die Bestimmung in einer Körperflüssigkeit eines Patienten auf immundiagnostischem Wege unter Verwendung geeigneter selektiver Antikörper unter praktischen Gesichtspunkten am vorteilhaftesten erscheint.

Aufgrund der Tatsache, dass bei einer experimentellen Sepsisauslösung erstmals das erhöhte Auftreten von CHP nachgewiesen werden konnte, wird somit die Möglichkeit geschaffen, CHP für diagnostische und/oder therapeutische Zwecke im Zusammenhang mit Entzündungen und Sepsis zu nutzen. Dafür können CHP oder geeignete Teilpeptide davon ggf. auch nach Verfahren, die inzwischen zum Stand der Technik gehören, synthetisch oder gentechnologisch gezielt hergestellt werden. CHP-Teilpeptide, ggf. in markierter Form, können auch als Kalibratoren, Tracer und Kompetitoren für bestimmte Assayformate für immundiagnostische Bestimmungen benötigt und dafür wie erläutert hergestellt werden.

Ferner können CHP-Fragmente bzw. geeignete Teilsequenzen davon nach bekannten Verfahren des modernen Standes der Technik auch zur Erzeugung spezifischer polyklonaler und insbesondere monoklonaler Antikörper verwendet werden, die als Hilfsmittel für die diagnostische Bestimmung von CHP in Körperflüssigkeiten eines Patienten und/oder auch als potentielle therapeutische Mittel geeignet sind. Die Erzeugung geeigneter monoklonaler Antikörper gegen bekannte Peptid-Teilsequenzen gehört heute zum allgemeinen Stand der Technik und muss nicht besonders beschrieben werden. Ferner ist auch ausdrücklich die Antikörpererzeugung unter Anwendung von Techniken der direkten genetischen Immunisierung mit einer entsprechenden DNA zu erwähnen. Es liegt somit im Rahmen der vorliegenden Erfindung, zur Immunisierung z.B. eine cDNA von CHP oder CHP-Fragmenten zu verwenden, da es sich in der Vergangenheit gezeigt hat, dass bei der Anwendung derartiger Immunisierungstechniken das Spektrum der gewinnbaren Antikörper erweitert werden kann. Es können aber auch bereits bekannte und von dritter Seite erhältliche Antikörper gegen CHP verwendet werden.

Bei der immunologischen Bestimmung von CHP kann dabei grundsätzlich so vorgegangen werden, wie das z.B. für die selektive Procalcitoninbestimmung beschrieben ist in P.P.Ghillani, et al., "Monoclonal antipeptide antibodies as tools to dissect closely related gene products", The Journal of Immunology, vol. 141, No.9, 1988, 3156-3163; und P.P.Ghillani, et al., "Identification and Measurement of Calcitonin Precursors in Serum of Patients with Malignant Diseases", Cancer research, vol.49, No.23, 1989, 6845-6851. Variationen der beschriebenen Techniken und/oder weitere Immunisierungstechniken kann der Fachmann einschlägigen Standardwerken und Veröffentlichungen entnehmen und sinngemäß anwenden.

CHP oder CHP-Fragmente mit den Teilsequenzen gemäß SEQ ID NO:1, SEQ ID NO:2, SEQ ID NO:4 und/oder SEQ ID NO:5 oder Teilpeptide davon, können aufgrund der vorliegenden Ergebnisse als spezifische Markerpeptide (Biomarker) zum diagnostischen Nachweis und zur Verlaufskontrolle von Entzündungen und Infektionen (insbesondere auch von systemischen Infektionen vom Sepsistyp) dienen. Wie die Bestimmung von Procalcitonin kann dabei die Bestimmung von CHP zur differentialdiagnostischen Früherkennung und zur Erkennung sowie für die Erstellung einer Verlaufsprognose, zur Beurteilung des Schweregrads und zur therapiebegleitenden Verlaufsbeurteilung von Sepsis und Infektionen erfolgen, wobei man bei einem derartigen Verfahren in einer Probe einer biologischen Flüssigkeit oder eines Gewebes eines Patienten den Gehalt von CHP bestimmt und aus der festgestellten Anwesenheit und/oder Menge von CHP auf das Vorliegen einer Entzündung, einer schweren Infektion oder einer Sepsis schließt und das erhaltene Ergebnis mit dem Schweregrad der Sepsis korreliert und die Behandlungsmöglichkeiten und/oder die Behandlungsaussichten abschätzt.

CHP (oder ggf. dafür kodierende DNA-Abschnitte) kann jedoch auch in der präventiven Medizin oder Therapie von Entzündungen, insbesondere von systemischen Entzündungen infektiöser Ätiologie und Sepsis, verwendet werden.

Da CHP eine immunsuppressive Wirkung hat, können die erhöhten Konzentrationen von CHP im Falle einer Sepsis als Gegenregulation des Körpers gegen die bei Sepsis beobachtete überschießende systemische Immunreaktion des Körpers gedeutet werden. Eine Unterstützung dieser Gegenreaktion durch externe CHP-Verabreichung sollte daher im Sinne einer Kontrolle der septischen Reaktion des Körpers wirken können, so dass der hierin berichtete diagnostische Befund erhöhter CHP-Konzentrationen bei Sepsis auch neue therapeutische Möglichkeiten erkennen läßt.

Die Beobachtung, dass die physiologischen CHP-Konzentrationen in Abhängigkeit von äußeren Stressreaktionen - im o.g. Tiermodell aufgrund von Toxineinwirkung - stark veränderlich sind, läßt ferner vermuten, dass derartige Veränderungen der Konzentrationen des endogenen Immunsuppressivums CHP auch in physiologischen Situationen auftreten, von denen bekannt ist, dass sie zu einer unerwünschten Schwächung der Immunabwehr führen, z.B. bei lokalen Entzündungen, die die Abwehr des gesamten Organismus schwächen, aber auch bei äußeren Stressbelastungen durch Überforderung oder bei seelischem Stress. In diesen Fällen ist die von CHP bewirkte Immunsuppression unerwünscht. Die therapeutische Beeinflussung erfolgt in solchen Fällen mit dem Ziel einer Absenkung der physiologischen CHP-Konzentrationen zur Regenerierung der Immunabwehr. Man kann in solchen Fällen die Konzentration von CHP durch Verabreichung von bindenden Antikörpern, oder durch extrakorporale Entfernung von CHP im Sinne einer Blutwäsche oder Plasmapherese mittels Affinitätsabsorption, vermindern. Für derartige Zwecke können auch Arzneimittel geschaffen werden, die als eigentlichen Wirkstoff gegen CHP oder CHP-Fragmente erzeugte, für eine Verabreichung an Patienten aufbereitete Antikörper zusammen mit einem geeigneten pharmazeutischen Träger enthalten.

Wird zu therapeutischen Zwecken die CHP-Wirkung verstärkt, sollen als therapeutisch verwendbare CHP-Materialien dabei auch solche Moleküle anzusehen sein, die CHP in unterschiedlicher posttranslational modifizierter Form, z.B. in glykosylierter oder phosphorylierter Form, oder auch in einer durch pharmazeutische Hilfsstoffe, z.B. Polyethylenglykolreste, substituierten Form enthalten.

Nachfolgend wird die Auffindung und Identifizierung von CHP in näheren Einzelheiten geschildert, wobei auf das beigefügte Sequenzprotokoll bezug genommen wird. Die Figuren zeigen:
- Fig. 1: Ansichten von 2D-Elektrophoresegelen, die einen Vergleich der Spotmuster von cytoplasmatischen Leberzellprotein eines gesunden Pavians (A) mit den Leberzellproteinen eines Pavians 5h nach einer durch LPS-Verabreichung induzierten Sepsis (B) ermöglichen. Der Pfeil zeigt die Position des erfindungsgemäßen sepsisspezifischen Produkts (CHP) an, das in Darstellung (B) durch einen Kreis hervorgehoben ist;

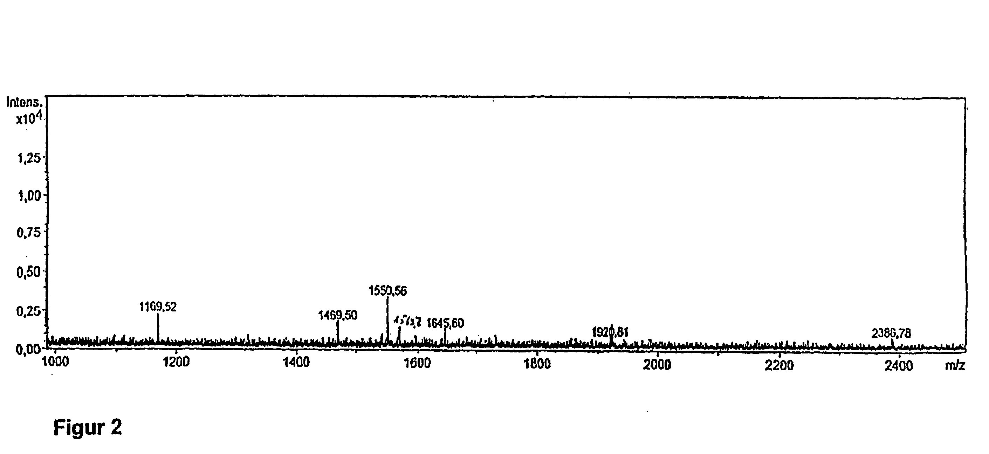

- Fig. 2: das Massenspektrum des aus dem Gel der 2D-Gelelektrophorese isolierten, trypsinverdauten Produkts.

### 1. Infektionssimmulation durch Endotoxinverabreichung im Tiermodell (Paviane).

In Anlehnung an die mit Pavianen durchgeführten Versuche zur Stimulierung der Procalcitonin-Ausschüttung durch Endotoxininjektionen (vgl.H.Redl, et al., "Procalcitonin release patterns in a baboon model of trauma and sepsis: Relationship to cytokines and neopterin", Crit Care Med 2000, Vol.28. No.11, 3659-3663; H.Redl, et al., "Non-Human Primate Models of Sepsis", in: Sepsis 1998; 2:243-253) wurden Pavianen (männlich, ca. 2 Jahre alt, 27 bis 29 kg schwer) jeweils 100 µg LPS (Lipopolysaccharid aus Salmonella Typhimurium, Bezugsquelle: Sigma) pro kg Körpergewicht intravenös verabreicht. 5 bis 5,5 h nach der Injektion wurden die Tiere durch intravenöse Verabreichung von 10 ml Doletal getötet. Innerhalb von 60 min nach ihrem Exitus wurden sämtliche Organe/Gewebe präpariert und durch Einfrieren in flüssigem Stickstoff stabilisiert.

Bei der weiteren Verarbeitung wurden Proben der einzelnen tiefgefrorenen Gewebe (1g) unter Stickstoffkühlung mit 1,5 ml Puffer A (50mM Tris/HCl, pH 7,1, 100mM KCl, 20% Glycerol) versetzt und in einem Porzellanmörser zu einem Mehl pulverisiert (vgl. J.Klose, "Fractionated Extraction of Total Tissue Proteins from Mouse and Human for 2-D Electrophoresis", in: Methods in Molecular Biology, Vol.112: 2-D Proteome Analysis Protocols, Humana Press Inc., Totowa, NJ).,Nach einer anschließenden 1-stündigen Zentrifugation bei 100.000 g und +4°C wurde der erhaltene Überstand gewonnen und bis zur weiteren Verarbeitung bei -80°C gelagert.

Unter Verwendung der auf diese Weise gewonnenen Gewebeextrakte wurde zuerst untersucht, in welchem der untersuchten Gewebe sich durch die Endotoxinverabreichung die höchsten Mengen des bekannten Sepsis-Biomarkers Procalcitonin erzeugen lassen. In dem ermittelten Gewebe mit der höchsten Procalcitoninbildung wurde dann mittels differentieller Proteomanalyse nach weiteren bisher nicht identifizierten proteinischen Produkten gesucht, die nur nach der Endotoxinverabreichung auftraten. Dazu wurden als Kontroll-Gewebeproben Gewebeproben unbehandelter Paviane verwendet, wobei die Tötung und Probengewinnung unter identischen Bedingungen erfolgte wie bei den behandelten Tieren.

### 2. Ermittlung von Paviangeweben mit der höchsten Procalcitoninbildung nach Endotoxininjektion.

Proben der einzelnen Gewebe wurden mit Hilfe eines Immunoluminometrischen Tests untersucht, der (in Anlehnung an den zur Bestimmung von humanem Procalcitonin entwickelten LU-MItest® PCT der Anmelderin) mit einem auf Polystyrolröhrchen immobilisierten Antikörper gegen Pavian-Calcitonin einerseits und einem mit einem Akridiniumester markierten monoklonalen Antikörper, der gegen den N-Terminus des Pavian-Procalcitonins gerichtet ist, arbeitet. Mit Hilfe dieses Tests wurden die Gehalte an Pavian-Procalcitonin in den einzelnen Proben nach Kalibrierung des Tests unter Verwendung von rekombinantem humanem Procalcitonin ermittelt.

Die Versuche ergaben, dass Lebergewebe die größte Procalcitoninmenge liefert. Für die Suche nach neuen sepsisspezifischen Biomarkern wurde daher mit den auf die eingangs beschriebene Weise gewonnenen Proteinextrakten aus Pavianleber gearbeitet.

### 3. Proteomanalyse unter Verwendung cytoplasmatischer Leberzellproteine von Pavianen.

Cytoplasmatische Leberzellproteinextrakte von einerseits gesunden Pavianen (Kontrolle) und andererseits Pavianen, denen LPS injiziert worden war, wurden im Rahmen einer Proteomanalyse verwendet. Bei der einleitenden analytischen 2D-Gelelektrophorese wurde Leberextrakt, 100 µg Protein enthaltend, auf 9M Harnstoff, 70 mM DTT, 2% Ampholyt pH 2-4 eingestellt und dann mittels analytischer 2D-Gelelektrophorese aufgetrennt, wie in J.Klose, et al., "Two-dimensional electrophoresis of proteins: An updated protocol and implications for a functional analysis of the genome", Electrophoresis 1995, 16, 1034-1059; beschrieben ist. Die Sichtbarmachung der Proteine im 2D-Elektrophoresegel erfolgte mittels Silverstaining (vgl. J.Heukeshoven, et al., "Improved silver staining procedure for fast staining in Phast-System Development Unit. I. Staining of sodium dodecyl gels", Electrophoresis 1988, 9, 28-32).

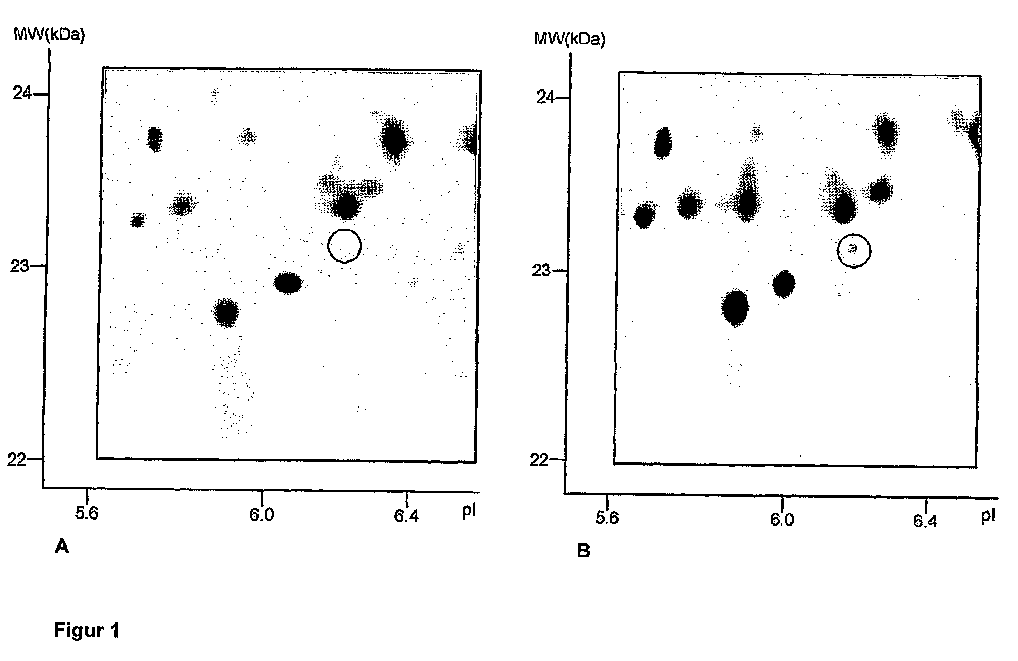

Zur Auswertung wurden die Proteinspotmuster der Proben unbehandelter Tieren mit den Proteinspotmustern verglichen, die aus Lebergewebeproben behandelter Tiere resultierten. Substanzen, die bei keiner Kontrollprobe, aber bei allen behandelten Tieren zusätzlich auftraten, wurden für weitere analytische Untersuchungen selektiert. Fig. 1 zeigt einen Vergleich der 2D-Elektrophoresegele für eine Kontrollprobe (A) und eine Probe eines behandelten Tieres (B), wobei der zusätzliche Proteinspot in (B) einem neuen Protein entspricht, dessen Position durch einen Pfeil und einen Kreis hervorgehoben ist.

Die im Proteinspotmuster der analytischen 2D-Gelelektrophorese identifizierten neuen spezifischen Proteine wurden dann anschließend mittels präparativer 2D-Gelelektrophorese unter Einsatz von 350 µg Protein präpariert (vgl. wiederum (10). Bei der präparativen 2D-Gelelektrophorese erfolgte die Färbung mittels Coomassie Brilliant Blue G250 (vgl. V.Neuhoff, et al., "Improved staining of proteins in polyacrylamide gels including isoelectric focusing gels with clear background at nanogram sensitivity using Coomassie Brilliant Blue G-250 and R-250", Electrophoresis 1988, 9, 255-262).

Die für die weitere Analyse vorselektierten Proteinspots wurden aus dem Gel ausgeschnitten, unter Anwendung der Methode, die in A.Otto, et al., "Identification of human myocardial proteins separated by two-dimensional electrophoresis using an effective sample preparation for mass spectrometry", Electrophoresis 1996, 17, 1643-1650; beschrieben ist, trypsinverdaut und anschließend massenspektroskopisch analysiert und zwar unter Anwendung massenspektrometrischer Untersuchungen, wie sie z.B. in G.Neubauer, et al., "Mass spectrometry and EST-database searching allows characterization of the multi-protein spliceosome complex", in: nature genetics vol. 20, 1998, 46-50; J.Lingner, et al., "Reverse Transcriptase Motifs in the Catalytic Subunit of Telomerase", in: Science, Vol.276, 1997, 561-567; M.Mann, et al., "Use of mass spectrometry-derived data to annotate nucleotide and protein sequence databases", in: TRENDS in Biochemical Sciences, Vol. 26, 1, 2001, 54-61; beschrieben und diskutiert werden. Dabei wurden die trypsinverdauten Proben nach einer ESI (ElectroSprayIonisierung) einer Tandem-Massenspektrometrie unterzogen. Es wurde ein Q-TOF-Massenspektrometer mit einer sog. nanoflow-Z-Spray-Ionenquelle der Firma Micromass, UK, verwendet. Dabei wurde entsprechend der Arbeitsanleitung des Geräteherstellers gearbeitet.

### 4. Identifizierung von CHP

Wie in den Figuren 1(A) und 1(B) gezeigt ist, findet sich in Leberzellextrakten von Pavianen, denen eine LPS-Injektion verabreicht worden war, u.a. ein Spot eines Proteins, für das aufgrund der Gelektrophoresedaten im Vergleich mit Markersubstanzen mit bekanntem Molekulargewicht ein Molekulargewicht von ca. 22 900 ± 700 Dalton abgeschätzt wurde, während aus der relativen Position des Proteins aus der ersten Dimension ein isoelektrischer Punkt von 6,1 bis 6,3 ermittelt wurde.

Bei der Massenanalyse des tryptischen Verdaus des neuen Proteinspots wurden Bruchstücke mit folgenden Massen (M/Z) gefunden: 1169,52; 1469,50; 1550,56; 1569,7; 1645,6; 1920,81; 2386,78. Zwei der 7 Massen (1169,52 und 1920,81) konnten keinen Massen bekannter trypsinbehandelter Proteine zugeordnet werden. Für die restlichen 5 Massen ergab sich die folgende Analyse und Zuordnung zu der bekannten Sequenz von CHP (vgl. SEQ ID NO:6; Nice Prot View of SWISS-PROT: Entry CA22_Human; Accession number Q99653; Lin X., Barber D.L. "A calcineurin homologous protein inhibits GTPase-stimulated na-H exchange", Proc.Natl.Acad.Sci.U.S.A. 93 :12636 (1996)) :

Die Masse 1469,50 entspricht der Sequenz L-Y-S-R-F-T-S-L-D-K (SEQ ID NO:1). An den Aminosäure S (Serin, 2faches Auftreten) und T (Threonin) ist die Sequenz durch jeweils eine Phosphatgruppe modifiziert. Das theoretische Molekulargewicht dieses Fragments beträgt 1469,499. In der Aminosäuresequenz von CHP (ohne den bei Lin X., supra, zusätzlich aufgeführten Methioninrest) findet sich diese Sequenz an den Positionen 31-40 wieder.

Die Masse 1550,56 entspricht der Sequenz K-E-T-G-F-S-H-S-Q-I-T (SEQ ID NO:2), ergänzt um 2 Phosphatgruppen. Das theoretische Molekulargewicht dieses Fragments beträgt 1550,564. In der Aminosäuresequenz von CHP (ohne den bei Lin X., supra, zusätzlich aufgeführten Methioninrest) findet sich diese Sequenz an den Positionen 19-30 wieder.

Die Masse 1569,7 entspricht der Sequenz T-L-A-H-F-R-P-I-E-D-N-E-K (SEQ ID NO:3). Das theoretische Molekulargewicht dieses Fragments beträgt 1569,71. In der Aminosäuresequenz von CHP (ohne den bei Lin X., supra, zusätzlich aufgeführten Methioninrest) findet sich diese Sequenz an den Positionen 86-98 wieder.

Die Masse 1645,6 entspricht der Sequenz A-S-T-L-L-R-D-E-E-I-K (SEQ ID NO:4). Das theoretische Molekulargewicht dieses Fragments beträgt 1645,597. In der Aminosäuresequenz von CHP (ohne den bei Lin X., supra, zusätzlich aufgeführten Methioninrest) findet sich diese Sequenz an den Positionen 5-18 wieder.

Die Masse 2386,78 entspricht der Sequenz I-I-N-A-F-F-P-E-G-E-D-Q-V-N-F-R-G-F-M-R (SEQ ID NO:5). Das theoretische Molekulargewicht dieses Fragments beträgt 2386,78. In der Aminosäuresequenz von CHP (ohne den bei Lin X., supra, zusätzlich aufgeführten Methioninrest) findet sich diese Sequenz an den Positionen 66-85 wieder.

Eine solche Übereinstimmung gilt nach anerkannten Interpretationsgrundsätzen als sichere Identifizierung des Produkts aus dem Proteinspot als CHP. Das Molekulargewicht von CHP (194 Aminosäuren) beträgt 22 325 Dalton.

Die erstmals festgestellten erhöhten CHP-Konzentrationen erlauben dessen Verwendung als neuer Biomarker für Sepsis.

## Patentansprüche

1. Verwendung des Calcineurin B Homologen Proteins (CHP) aus Körperflüssigkeiten oder Körpergeweben als Markerpeptid in *in vitro*-Verfahren zum diagnostischen Nachweis, für die Verlaufsprognose und für die Verlaufskontrolle von Sepsis.

2. Verwendung von CHP nach Anspruch 1 im Rahmen der differentialdiagnostischen Früherkennung und Erkennung für die Verlaufsprognose, die Beurteilung des Schweregrads und für die therapiebegleitende Verlaufsbeurteilung von Sepsis durch Bestimmung des Auftretens und/oder der Menge von CHP in einer biologischen Flüssigkeit oder einer Gewebeprobe eines Patienten.

3. *In vitro*-Verfahren zur differentialdiagnostischen Früherkennung und Erkennung, für die Verlaufsprognose und die Beurteilung des Schweregrads und zur therapiebegleitenden Verlaufsbeurteilung von Sepsis, **dadurch gekennzeichnet, dass** man die Anwesenheit und/oder Menge von CHP in einer biologischen Flüssigkeit oder einer Gewebeprobe eines Patienten bestimmt und aus der bestimmten Anwesenheit und/oder Menge von CHP Schlüsse hinsichtlich des Vorliegens, des zu erwartenden Verlaufs, des Schweregrads oder den Erfolg eines Therapie der Sepsis zieht.

4. *In vitro*-Verfahren nach Anspruch 3, **dadurch gekennzeichnet, dass** es ein immundiagnostisches Bestimmungsverfahren ist.

## Claims

1. Use of calcineurin B homologous protein (CHP) from body fluids or body tissues as a marker peptide in *in vitro* methods for diagnosis, for prognosis and for monitoring of the course of sepsis.

2. Use of CHP according to Claim 1 in the differential early diagnosis and diagnosis for prognosis, for assessment of the severity and for the therapy-accompanying assessment of the course of sepsis by determination of the occurrence and/or the amount of CHP in a biological fluid or tissue sample of a patient.

3. *In vitro* method for differential early diagnosis and diagnosis, for prognosis and for the assessment of the severity and for the therapy-accompanying assessment of the course of sepsis, **characterized in that** the presence and/or amount of CHP in a biological fluid or a tissue sample of a patient is determined and conclusions with regard to the presence, the expected course, the severity or the success of the therapy of sepsis are drawn from the determined presence and/or amount of CHP.

4. Method according to Claim 3, **characterized in that** it is an immunodiagnostic method of determination.

## Revendications

1. Utilisation de la protéine calcineurine B homologène (CHP) de liquides corporels ou de tissus corporels comme peptide de marquage dans des procédés *invitro* de détection de diagnostic pour pronostiquer et contrôler l'évolution de la septicémie.

2. Utilisation de la CHP selon la revendication 1 dans le cadre de la reconnaissance précoce par diagnostic différentiel et de la reconnaissance par diagnostic différentiel pour pronostiquer l'évolution de la septicémie, en évaluer le degré de gravité et en évaluer l'évolution au cours de la thérapie, par la détermination de la présence et/ou de la quantité de CHP dans un liquide biologique ou dans un échantillon de tissu d'un patient.

3. Procédé in *vitro* de reconnaissance par diagnostic différentiel et de reconnaissance précoce par diagnostic différentiel pour pronostiquer l'évolution de la septicémie, en évaluer le degré de gravité et en évaluer l'évolution au cours de la thérapie, **caractérisé en ce que** l'on détermine la présence et/ou la quantité de CHP dans un liquide biologique ou dans un échantillon de tissu d'un patient et, sur base de la présence et/ou de la quantité de CHP déterminé, on tire des conclusions relatives à la présence de la septicémie, à son évolution probable, à son degré de gravité ou au résultat d'une thérapie.

4. Procédé *in vitro* selon la revendication 3, **caractérisé en ce qu'**il s'agit d'un procédé de détermination par immunodiagnostic.
